# EUROPEAN PATENT SPECIFICATION

(11) **EP 1 244 384 B1**
(45) Date of publication and mention of the grant of the patent: **04.06.2008**
(21) Application number: 99965547.5
(22) Date of filing: 23.12.1999
(51) Int. Cl.: A61B 5/117

(54) **PATIENT-IDENTIFIED MEASURING**
SYSTEM ZUR MESSUNG VON PARAMETER IDENTIFIZIERTEN PATIENTEN
MESURE AVEC IDENTIFICATION DU PATIENT

(43) Date of publication of application: 02.10.2002
(73) Proprietor: Philips Intellectual Property & Standards GmbH, 20099 Hamburg (DE)
(72) Inventor: WESTERTEICHER, Christoph, D-71229 Leonberg (DE)
(74) Representative: Volmer, Georg
(86) International application number: PCT/EP1999/010346
(87) International publication number: WO 2001/047418

(56) References cited:
- WO-A-00/28460
- WO-A-99/07282
- WO-A-99/18532

## Description

### BACKGROUND OF THE INVENTION

The present invention relates to the measurement of a physiological parameter of a patient.

Most western countries face the issue of needing to contain health care expenditure, whilst at the same time there is a growing number of elderly and chronically ill people who potentially will burden the health care system in the years ahead. Policy is geared toward stimulating better use of existing services and reengineering delivery of care.

One very promising solution is the transfer of services from hospital to out-of-hospital settings, e.g. at home or in remote locations using Telemedicine solutions. Telemedicine is defined as "medicine at a distance", thus enabling care providers to diagnose and treat patients at their point of need. Technically the Telemedicine solution can consist of monitoring devices deployed in the patients home. These measure vital signs, and transmit the recorded data via existing media (e.g. the phone line) to a care provider.

It is this separation of care provider and patient, that introduces the requirement to unambiguously assign a recorded measurement signal to a specific individual. Since the care provider does not see the person conducting the measurement, false readings and/or interpretations of the recorded measurement signals are possible. This problem becomes even more serve when the same measurement devices are used, intentionally or accidentally, by more than one patient. Again, the measured data have to be clearly assigned to a specific individual.

By WO 99/18532 a network system for remote monitoring individuals is known. The system comprises the features of the first part of claims 1 and 8 and includes a server and a remote interface for entering in the server a set of queries to be answered by the individual. The server includes a database comprising a look-up table. The look-up table contains a list of patients to be monitored and for each patient a unique patient identification code. Furthermore the look-up table comprises pointer to a script program assigned to the patient. Each remote apparatus is designed to execute assigned script programs, which it receives from the server and transmit the responses and measurements to the server.

By WO 99/07282 a method and apparatus for collecting medical data from a test subject while optionally preserving anonymity for the test subject is known. For the identification biometric data from the test subject is taken, for example a finger print, iris data or hand geometry. Preferable the biometric data is taken simultaneously with collecting the sample from the subject.

In a known solution, a correlation of the measured patient vital signs with a previously recorded data is provided for assigning recorded measurement signals to a specific individual. This concept takes advantage of the fact that normally physiological parameters only change within specific limits. Data recorded exhibiting more dramatic fluctuations than physiologically possible can thus clearly be detected and eliminated, because they cannot stem from the patient. This concept, however, does not support the recognition of erroneous vital sign measurements that are within the physiological range of the patient, but not generated by that patient. The solution is dependent on the availability of (previous) baseline data, and also does not allow numerous patients to intentionally use the same devices.

### SUMMARY OF THE INVENTION

It is an object of the invention to unambiguously assign a recorded measurement signal to a specific individual. The object is solved by the independent claims. Preferred embodiments are shown by the dependent claims.

According to the invention, a measurement signal of a specific individual is provided with an identification signal for clearly identifying the specific person performing the measurement. The patient identification signal provides an unambiguous identifier, allowing remote recognition of the individual performing the measurement. The patient identification signal can preferably be provided e.g. by:
● a user-initiated entry of a specific identifier (e.g. key-code, smart cards),
● an automatic sensing e.g. of tags worn by the patient,
● an evaluation of characteristic biological features (e.g. fingerprint, voice).

For combining/associating (or linking) the identification signal with the measurement signal, the measurement equipment comprises a patient identification reader (PIR). In a close temporal relationship to the actual physiological signal measuring process, e.g. during the measuring process or (shortly) before or after the measuring process, however, at least before a succeeding measuring process, the patient will be identified, and the PIR can provide the patient identification to the measurement equipment, which again can associate the identification signal with the measurement signal. The patient can be identified by sending the identification signal, or any kind of identifier from which the identification signal can be derived from, to the PIR.

The PIR might probe whether the person performing the current measurement has a valid identification. Preferably only when the PIR recognizes a valid identifier, it will (electronically) attach the identification signal to the patient's measured parameter(s). Thus, each patient signal measured can be explicitly assigned to a specific patient. Alternatively, if the person performing the measurement has no valid identifier, the measurement can be discarded.

In a first aspect of the invention, the identification signal will be associated with the measurement signal by an active (preferably user initiated) interaction of the individual or a third person. In a preferred embodiment, patients are required to enter the identification signal or an identifier from which the identification signal can be derived, such as a key code or a smart card, e.g. either before or after conducting a measurement. This requires that the respective measurement device is equipped with some form of key-code or a smart card reader interface. Additionally, it demands significant patient interaction, by having to remember the correct key-code, as well as the sequence in which all these activities (physiologic measurement and key-code entry) have to be performed. Potentially, this mechanism of identifying the patients excludes specific groups of users, such as people with mental disabilities.

In a second aspect of the invention, the identification signal will be automatically associated with the measurement signal. The measurement equipment comprises the patient identification reader PIR, and the patient carries or is equipped (e.g. in form of a bracelet or clip) with a corresponding patient identification device (PID). The PIR and the PID are adapted to establish a communication therebetween, at least in a close temporal relationship to the actual physiological signal measuring process.

During the communication, the PID identifies the patient, and the PIR can provide the patient identification to the measurement equipment, which again can associate the identification signal with the measurement signal. The PID can identify the patient by sending the identification signal, or any kind of identifier from which the identification signal can be derived from, to the PIR. In a preferred embodiment, the

PIR sends out an electrical signal or scans (e.g. barcode, fingerprint etc.) the PID in chronological context with the physiological measurement.

The communication between the patient identification device PID and the patient identification reader PIR can be provided by any means as known in the art, such as wired or wireless transmission (e.g. using ultrasound, infrared, magnetic, radio frequency, optical, or other electro-magnetic transmission types).

The PIR might then probe whether the person performing the current measurement has a valid identification. When the PIR recognizes an eligible PID, it registers its identifier (e.g. a unique identification number), and electronically attaches the identification signal (e.g. this unambiguous identification number) to the patient's measured parameter(s).

In a preferred embodiment, the PIR comprises a sensing device capable of reading the identifier from the PID and combining the data recorded by the physiological measurement equipment and the unique person identification to an information package that is clearly assigned to one specific individual.

The sensing device can be implemented utilizing a variety of technologies, ranging from optical solutions (e.g. barcode readers, infra-red), wireless electrical concepts (e.g. RF transponders, magnetic coupling), to bio-sensing devices e.g. capable of analyzing the patient's finger print, voice characteristics or other unique biological markers. In the latter case the patients personal characteristics (e.g. fingerprint, voice etc.) are utilized as PIDs to correlate physiological measurements to specific individuals.

The PIR can be an integral part of the measurement equipment or only connected thereto. The PIR preferably probes if the individual performing a measurement has a valid identifier of the PID, and if recognition is positive, links this recorded identification with the measured signal.

In a preferred embodiment of an Automatic Patient Identification System (APIS), the PID comprises a compact transponder that carries a tamper-proof identification number. This transponder can be packaged in a variety of sizes. It is preferably ultra thin and battery free, allowing it to be worn by the patient, for example as a bracelet or a clip. When the patient performs a measurement, the PIR of the measuring device automatically interrogates the user's transponder e.g. by a radio frequency (RF) reader. This implies that the transponder does not have to be in direct mechanical contact with the measurement device. The patient, however, needs to be in proximity to the PIR for the reader to link up with the transponder. If the measurement unit detects a valid transponder it transmits the identification signal (e.g. an identification number) together with the measured vital signs to the care provider. Instead of using an RF link between the transponder and the reader, this concept could also be implemented using e.g. magnetic coupling.

This solution requires no intervention by the patient other than wearing the transponder, and initiation of the measurement. Interrogation of eligible users and transmission of the recorded identification, as well as the patients measured physiological parameters is a totally automatic process performed by the measurement unit. It is clear that the invention can be partly or entirely embodied by one or more suitable software programs, which can be stored on or otherwise provided by any kind of data carrier, and which might be executed in or by any suitable data processing unit.

This concept allows physiological measurements to be clearly assigned to one individual. It also enables filtering of measurements performed by individuals with valid or no identifier. Additionally, numerous people (patients) can use the same set of measurement devices, by electronically tagging each recorded physiological signal, linking it to one specific person.

It is clear that the invention can be partly or entirely embodied by one or more suitable software programs, which can be stored on or otherwise provided by any kind of data carrier, and which might be executed in or by any suitable data processing unit.

### BRIEF DESCRIPTION OF THE DRAWINGS

Other objects and many of the attendant advantages of the present invention will be readily appreciated and become better understood by reference to the following detailed description when considering in connection with the accompanied drawings. Features that are substantially or functionally equal or similar will be referred to with the same reference sign(s).

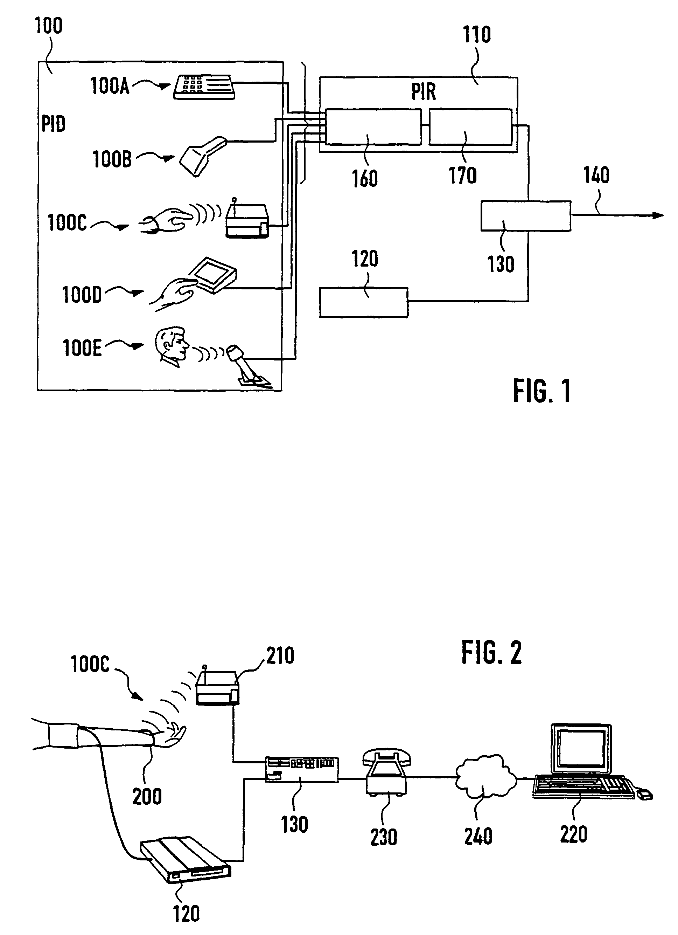

- Figs. 1 and 2: depict an Automatic Patient Identification System (APIS) as an example of an embodiment according to the present invention.

### DETAILED DESCRIPTION OF THE INVENTION

Fig. 1 depicts an Automatic Patient Identification System (APIS) as an example of an inventive embodiment. A Patient Identification Device (PID) 100 is coupled with a Patient Identification Reader (PIR) 110 for identifying a patient's measuring results provided by a physiological measurement unit 120. For clearly identifying the specific person performing the measurement, the PID 100 provides an unambiguous identifier for the patient to the PIR 110. The PIR 110, again, checks the identifier from the PID 100 and sends a patient identification signal to a signal packaging unit 130 which further receives the patient's measuring results from the physiological measurement unit 120. The signal packaging unit 130 combines the patient's measuring results with the patient identification signal to a combined measurement and ID output signal 140.

Fig. 1 shows a plurality of different solution for the PID 100, which can be applied either individually or in combination. PID 100A represents a keypad entry for manually typing in a patient identifier, such as a PIN code. PID 100B represents a barcode solution for reading a barcode or the like containing a patient identifier. PID 100C shows a transponder concept, wherein a patient identifier is registered via an RF link between a transponder worn or otherwise held by the patient and receiving unit (which might also be part of the PIR 110). PIDs 100D and 100E represent identification systems based on identification by physical properties of the patient, such as fingerprint (100D) or voice (100E).

The PIR 110 preferably comprises a reading unit 160 for reading the identifier from the PID 100 and an identifier evaluation unit 170 for validating the identifier.

Fig. 2 depicts a more conceptual view of the Automatic Patient Identification System (APIS) as depicted in Fig.1 for the example of the PID 100C. The PID 100C comprises a transponder 200 worn e.g. at the patient's arm and a transmission unit 210, which is preferably integrated in the PIR 110, for establishing a radio frequency communication therebetween. The transmission unit 210 is equipped with a radio frequency transmitter/receiver and an antenna. Before or during a measurement, the PIR 130 sends out a signal (via the transmission unit 210) probing if the person performing the measurement is wearing an eligible transponder 200. The low energy signal transmitted by the transmission unit 210 activates the transponder 200, thus the transponder 200 does not need to be powered by itself (e.g. by a battery). The activation signal from the transmission unit 210 also instructs the transponder 200 to transmit back its stored patient identifier, such as an identification number.

The reading unit 160 reads out the patient identifier from the transmission unit 210. If the PIR 110 comprises the identifier evaluation unit 170, it will validate the patient identifier whether it is eligible. If the patient identifier is eligible, the PIR 110 sends an identification signal derived from the patient identifier to the signal packaging unit 130. The signal packaging unit 130, which further receives the patient's measuring results from the physiological measurement unit 120, combines both signals and sends the combination to a remote user 220 (e.g. a reviewing clinician), preferably via a modem 230 and a telecommunication system 240. Alternatively, the signal packaging unit 130 may simply package the identification signal and the measured signal and transmits this information to the remote user 220.

## Claims

1. A device for providing a patient-identified measuring signal (140), comprising:
a means (130) adapted for receiving from a measuring device (120) a measuring signal representative for a physiological parameter of a patient,
a patient identification reader (110) for receiving from a patient identification device (100) an identification signal identifying the patient, and
a means (130) for combining and/or associating the measured signal with the identification signal for providing the patient-identified measuring signal (140),
**characterised in that**
the patient identification reader (110) is adapted for validating whether the identification signal is eligible, wherein only when the patient identification reader (110) recognizes an eligible identification signal, the identification signal is attached to the measured signal.

2. A system for measuring a physiological parameter of a patient, comprising:
the device according to claim 1,
a measuring device (120) for measuring the signal representative for the physiological parameter of the patient, and
a patient identification device (100) for providing the identification signal for identifying the patient.

3. The system of claim 2, wherein the patient identification device (100) comprises:
a means for providing the identification signal by an active interaction of the patient or a third person, preferably by entering the identification signal or an identifier from which the identification signal can be derived.

4. The system of claim 2, wherein the patient identification device (100) comprises:
a means for automatically providing the identification signal without requiring an active interaction of the patient or a third person, preferably by automatically reading out the identification signal, or an identifier from which the identification signal can be derived, from a patient identification device preferably carried by the patient.

5. The system of claim 4, wherein the patient identification device (100) comprises:
a means for establishing a communication with the patient identification reader (110) for reading out the identification signal, or an identifier from which the identification signal can be derived.

6. The system of any one of claims 1 to 5, wherein the patient identification device (100) comprises:
a means (100A) adapted for receiving a user initiated entry of the identification signal or a corresponding identifier, preferably a key-code or a smart card, and/or
a means (100C) for automatically sensing the identification signal or a corresponding identifier, preferably by means of a tag and/or a transponder worn or otherwise carried by the patient, and/or
a means (100D, 100E) for evaluating the characteristic of a biological feature of the patient, preferably by a fingerprint and/or the voice.

7. The system of any one of claims 1 to 6, wherein the patient identification reader (110) comprises:
a reader unit (160) for establishing a communication with the patient identification device (100) for reading out the identification signal or a corresponding identifier therefrom.

8. A method for providing a patient-identified measuring signal (140), comprising the following steps:
receiving a measuring signal representative for a physiological parameter of a patient,
receiving an identification signal identifying the patient from a patient identification device (100), and
combining and/or associating the measured signal with the identification signal for providing the patient-identified measuring signal (140),
**characterised by**
validating whether the identification signal is eligible, and
attaching the identification signal to the measured signal only when an eligible identification signal is identified.

9. The method of claim 8, wherein the step of receiving the identification signal is executed in a close temporal relationship to the step of receiving the measuring signal representative for the physiological parameter of the patient, preferably during the measuring process for the physiological parameter of the patient or before or after the measuring process.

10. The method of claim 9, wherein the step of receiving the identification signal is executed before a succeeding measuring process

11. The method of any of claims 8 to 10, wherein the identification signal is provided by an active interaction of the patient or a third person, preferably by entering the identification signal or an identifier from which the identification signal can be derived.

12. The of any of claims 8 to 10, wherein the identification signal is automatically provided without requiring an active interaction of the patient or a third person, preferably by automatically reading out the identification signal, or an identifier from which the identification signal can be derived, from a patient identification device preferably carried by the patient.

13. The method of claim 12, wherein a communication is established for reading out the identification signal, or an identifier from which the identification signal can be derived.

## Patentansprüche

1. Vorrichtung zum Bereitstellen eines den Patienten identifizierenden Messsignals (140), die Folgendes umfasst:
ein Mittel (130), das dafür eingerichtet ist, von einer Messvorrichtung (120) ein Messsignal zu empfangen, das einen physiologischen Parameter eines Patienten darstellt,
einen Patientenidentifikationsleser (110) zum Empfangen eines den Patienten identifizierenden Identifikationssignals von einer Patientenidentifikationsvorrichtung (100), und
ein Mittel (130), um das gemessene Signal mit dem Identifikationssignal zu kombinieren und/oder diesem zuzuordnen, um das den Patienten identifizierende Messsignal (140) zu liefern,
**dadurch gekennzeichnet, dass**
der Patientenidentifikationsleser (110) dafür eingerichtet ist, zu validieren, ob das Identifikationssignal zulässig ist, wobei das Identifikationssignal nur dann an das gemessene Signal angehängt wird, wenn der Patientenidentifikationsleser (110) ein zulässiges Identifikationssignal erkennt.

2. System zum Messen eines physiologischen Parameters eines Patienten, das Folgendes umfasst:
die Vorrichtung nach Anspruch 1,
eine Messvorrichtung (120) zum Messen des für den physiologischen Parameter des Patienten repräsentativen Signals, und
eine Patientenidentifikationsvorrichtung (100) zum Bereitstellen des Identifikationssignals zum Identifizieren des Patienten.

3. System nach Anspruch 2, wobei die Patientenidentifikationsvorrichtung (100) Folgendes umfasst:
ein Mittel zum Bereitstellen des Identifikationssignals durch einen aktiven Eingriff des Patienten oder einer dritten Person, vorzugsweise durch Eingabe des Identifikationssignals oder eines Identifikators, von dem das Identifikationssignal abgeleitet werden kann.

4. System nach Anspruch 2, wobei die Patientenidentifikationsvorrichtung (100) Folgendes umfasst:
ein Mittel zum automatischen Bereitstellen des Identifikationssignals ohne dass ein aktiver Eingriff von Seiten des Patienten oder einer dritten Person erforderlich ist, vorzugsweise durch automatisches Auslesen des Identifikationssignals oder eines Identifikators, von dem das Identifikationssignal abgeleitet werden kann, aus einer Patientenidentifikationsvorrichtung, die vorzugsweise durch den Patienten getragen wird.

5. System nach Anspruch 4, wobei die Patientenidentifikationsvorrichtung (100) Folgendes umfasst:
ein Mittel zum Aufbauen einer Kommunikationsverbindung mit dem Patientenidentifikationsleser (110) zum Auslesen des Identifikationssignals oder eines Identifikators, von dem das Identifikationssignal abgeleitet werden kann.

6. System nach einem der Ansprüche 1 bis 5, wobei die Patientenidentifikationsvorrichtung (100) Folgendes umfasst:
ein Mittel (100A), das dafür eingerichtet ist, eine vom Benutzer eingeleitete Eingabe des Identifikationssignals oder eines entsprechenden Identifikators, vorzugsweise eines Schlüsselcodes oder einer Smart Card, zu empfangen, und/oder
ein Mittel (100C) zum automatischen Erkennen des Identifikationssignals oder eines entsprechenden Identifikators, vorzugsweise mit Hilfe eines Anhängers und/oder eines Transponders, der von dem Patienten getragen oder auf andere Weise mitgeführt wird, und/oder
ein Mittel (100D, 100E) zum Evaluieren der Eigenschaft eines biologischen Merkmals des Patienten, vorzugsweise durch einen Fingerabdruck und/oder die Stimme.

7. System nach einem der Ansprüche 1 bis 6, wobei der Patientenidentifikationsleser (110) Folgendes umfasst:
eine Leseeinheit (160) zum Aufbauen einer Kommunikationsverbindung mit der Patientenidentifikationsvorrichtung (100), um das Identifikationssignal oder einen entsprechenden Identifikator daraus auszulesen.

8. Verfahren zum Bereitstellen eines den Patienten identifizierenden Messsignals (140), das die folgenden Schritte umfasst:
Empfangen eines Messsignals, das einen physiologischen Parameter eines Patienten darstellt,
Empfangen eines den Patienten identifizierenden Identifikationssignals von einer Patientenidentifikationsvorrichtung (100), und
Kombinieren des gemessenen Signals mit dem Identifikationssignal und/oder Zuordnen des gemessenen Signals zu dem Identifikationssignal, um das den Patienten identifizierende Messsignal (140) zu liefern,
**gekennzeichnet durch**
das Validieren, ob das Identifikationssignal zulässig ist, und
das Anhängen des Identifikationssignals an das gemessene Signal nur dann, wenn ein zulässiges Identifikationssignal erkannt wurde.

9. Verfahren nach Anspruch 8, wobei der Schritt des Empfangens des Identifikationssignals in engem zeitlichen Zusammenhang mit dem Schritt des Empfangens des den physiologischen Parameter des Patienten darstellenden Messsignals ausgeführt wird, vorzugsweise während des Messvorgangs zum Erfassen des physiologischen Parameters des Patienten oder vor oder nach dem Messvorgang.

10. Verfahren nach Anspruch 9, wobei der Schritt des Empfangens des Identifikationssignals vor einem nachfolgenden Messvorgang ausgeführt wird.

11. Verfahren nach einem der Ansprüche 8 bis 10, wobei das Identifikationssignal durch einen aktiven Eingriff des Patienten oder einer dritten Person bereitgestellt wird, vorzugsweise durch Eingabe des Identifikationssignals oder eines Identifikators, von dem das Identifikationssignal abgeleitet werden kann.

12. Verfahren nach einem der Ansprüche 8 bis 10, wobei das Identifikationssignal automatisch bereitgestellt wird, ohne dass ein aktiver Eingriff von Seiten des Patienten oder einer dritten Person erforderlich ist, vorzugsweise durch automatisches Auslesen des Identifikationssignals oder eines Identifikators, von dem das Identifikationssignal abgeleitet werden kann, aus einer Patientenidentifikationsvorrichtung, die vorzugsweise durch den Patienten getragen wird.

13. Verfahren nach Anspruch 12, wobei eine Kommunikationsverbindung zum Auslesen des Identifikationssignals oder eines Identifikators, von dem das Identifikationssignal abgeleitet werden kann, aufgebaut wird.

## Revendications

1. Dispositif destiné à fournir un signal de mesure avec identification de patient (140), comprenant:
un moyen (130) propre à recevoir d'un dispositif de mesure (120) un signal de mesure représentatif d'un paramètre physiologique d'un patient,
un lecteur d'identification de patient (110) pour recevoir d'un dispositif d'identification de patient (100) un signal d'identification identifiant le patient, et
un moyen (130) pour combiner et/ou associer le signal mesuré avec le signal d'identification afin de fournir le signal de mesure avec identification de patient (140),
**caractérisé en ce que**
le lecteur d'identification de patient (110) est susceptible de valider si le signal d'identification est acceptable, sachant que ce n'est que lorsque le lecteur d'identification de patient (110) reconnaît un signal d'identification acceptable, que le signal d'identification est attaché au signal mesuré.

2. Système pour mesurer un paramètre physiologique d'un patient, comprenant:
le dispositif suivant la revendication 1,
un dispositif de mesure (120) pour mesurer le signal représentatif du paramètre physiologique du patient, et
un dispositif d'identification de patient (100) pour fournir le signal d'identification destiné à identifier le patient.

3. Système suivant la revendication 2, dans lequel le dispositif d'identification de patient (100) comprend:
un moyen pour fournir le signal d'identification par une interaction active du patient ou d'une tierce personne, de préférence en introduisant le signal d'identification ou un identifiant duquel le signal d'identification peut être dérivé.

4. Système suivant la revendication 2, dans lequel le dispositif d'identification de patient (100) comprend:
un moyen pour fournir automatiquement le signal d'identification sans exiger une interaction active du patient ou d'une tierce personne, de préférence en lisant automatiquement le signal d'identification, ou un identifiant duquel le signal d'identification peut être dérivé, à partir d'un dispositif d'identification de patient porté de préférence par le patient.

5. Système suivant la revendication 4, dans lequel le dispositif d'identification de patient (100) comprend:
un moyen pour établir une communication avec le lecteur d'identification de patient (110) afin de lire le signal d'identification, ou un identifiant à partir duquel le signal d'identification peut être dérivé.

6. Système suivant l'une quelconque des revendications 1 à 5, dans lequel le dispositif d'identification de patient (100) comprend:
un moyen (100A) propre à recevoir une entrée, déclenchée par l'utilisateur, du signal d'identification ou d'un identifiant correspondant, de préférence un code clé ou une carte à puce, et/ou
un moyen (100C) pour détecter automatiquement le signal d'identification ou un identifiant correspondant, de préférence au moyen d'une étiquette et/ou d'un transpondeur porté ou autrement véhiculé par le patient, et/ou
un moyen (100D, 100E) pour évaluer la caractéristique d'une particularité biologique du patient, de préférence par une empreinte digitale et/ou la voix.

7. Système suivant l'une quelconque des revendications 1 à 6, dans lequel le lecteur d'identification de patient (110) comprend:
une unité de lecteur (160) pour établir une communication avec le dispositif d'identification de patient (100) afin de lire le signal d'identification ou un identifiant correspondant à partir de celui-ci.

8. Procédé pour fournir un signal de mesure avec identification de patient (140) comprenant les étapes suivantes:
réception d'un signal de mesure représentatif d'un paramètre physiologique d'un patient,
réception d'un signal d'identification identifiant le patient à partir d'un dispositif d'identification de patient (100), et
combinaison et/ou association du signal mesuré avec le signal d'identification pour fournir le signal de mesure avec identification du patient (140),
**caractérisé par**
une validation si le signal d'identification est acceptable, et
l'union du signal d'identification au signal mesuré uniquement lorsqu'un signal d'identification acceptable est identifié.

9. Procédé suivant la revendication 8, dans lequel l'étape de réception du signal d'identification est exécutée dans une relation temporelle étroite avec l'étape de réception du signal de mesure représentatif du paramètre physiologique du patient, de préférence pendant le processus de mesure du paramètre physiologique du patient ou avant ou après le processus de mesure.

10. Procédé suivant la revendication 9, dans lequel l'étape de réception du signal d'identification est exécutée avant un processus de mesure suivant.

11. Procédé suivant l'une quelconque des revendications 8 à 10, dans lequel le signal d'identification est fourni par une interaction active du patient ou d'une tierce personne, de préférence en entrant le signal d'identification ou un identifiant à partir duquel le signal d'identification peut être dérivé.

12. Procédé suivant l'une quelconque des revendications 8 à 10, dans lequel le signal d'identification est fourni automatiquement sans exiger une interaction active du patient ou d'une tierce personne, de préférence en lisant automatiquement le signal d'identification, ou un identifiant à partir duquel le signal d'identification peut être dérivé, d'un dispositif d'identification de patient porté de préférence par le patient.

13. Procédé suivant la revendication 12, dans lequel une communication est établie pour lire le signal d'identification, ou un identifiant à partir duquel le signal d'identification peut être dérivé.
